(19) Europäisches Patentamt
European Patent Office
Office européen des brevets

(11) Veröffentlichungsnummer: **0 296 592 B1**

(12) # EUROPÄISCHE PATENTSCHRIFT

(45) Veröffentlichungstag der Patentschrift: **12.02.92**

(51) Int. Cl.$^5$: **C07C 215/10**, C07D 295/08, A61K 31/13, A61K 31/395

(21) Anmeldenummer: **88110033.3**

(22) Anmeldetag: **23.06.88**

Die Akte enthält technische Angaben, die nach dem Eingang der Anmeldung eingereicht wurden und die nicht in dieser Patentschrift enthalten sind.

(54) **Aminopropanolderivate von 3-(2-Hydroxyphenyl)-1-propanon-Verbindungen, Verfahren zu ihrer Herstellung und diese Verbindungen enthaltende Arzneimittel.**

(30) Priorität: **23.06.87 EP 87109015**

(43) Veröffentlichungstag der Anmeldung: **28.12.88 Patentblatt 88/52**

(45) Bekanntmachung des Hinweises auf die Patenterteilung: **12.02.92 Patentblatt 92/07**

(84) Benannte Vertragsstaaten: **AT BE CH DE ES FR GB GR IT LI LU NL SE**

(56) Entgegenhaltungen:
**EP-A- 0 075 207**
**FR-A- 2 362 115**
**US-A- 4 540 697**

(73) Patentinhaber: **Helopharm W. Petrik GmbH & Co.KG.**
**Waldstrasse 23-25**
**W-1000 Berlin 51(DE)**

(72) Erfinder: **Petrik, Gerd**
**Herthastrasse 11**
**W-1000 Berlin 33(DE)**
Erfinder: **Schubert,Klemens, Dr.**
**Eichborndamm 5**
**W-1000 Berlin 51(DE)**

(74) Vertreter: **Vossius & Partner**
**Siebertstrasse 4 P.O. Box 86 07 67**
**W-8000 München 86(DE)**

**EP 0 296 592 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Aminopropanolderivate von 3-(2'-Hydroxyphenyl)-1-propanon-Verbindungen und ihre physiologisch verträglichen Salze mit Säuren, Verfahren zu ihrer Herstellung sowie diese Verbindungen enthaltende Arzneimittel.

Aus der DE-PS 2001431 sind 2-(2'-Hydroxy-3'-alkylaminopropoxy)-$\beta$-phenylpropiophenone der allgemeinen Formel

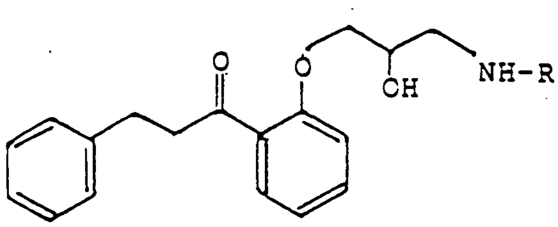

sowie deren Säureadditionssalze bekannt. Die n-Propylaminoverbindung (R = n - $C_3H_7$ - Propafenon) weist eine antiarrhythmische Wirksamkeit auf.

Aus der EP-A-0 074 014 sind das 2-[2'-Hydroxy-3'-(1,1-dimethylpropylamino)-propoxy]-$\beta$-phenylpropiophenon (Diprafenon) und seine Säureadditionssalze bekannt. Diprafenon ist ein Antiarrhythmikum.

Ferner sind aus der EP-A-0 075 207 Aminopropanolderivate der allgemeinen Formel

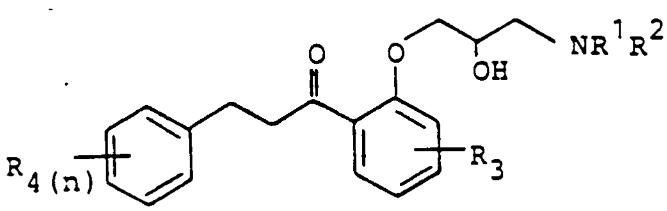

und deren Salze bekannt.

Typische Beispiele für $R^1$ - $R^4$ sind Wasserstoffatome oder Alkylreste; n hat einen Wert von 1, 2 oder 3.

Die vorstehend genannten Verbindungen sind Antiarrhythmika.

Aufgabe der vorliegenden Erfindung ist es, neue Aminopropanolderivate von 3-(2'-Hydroxyphenyl)-1-propanon-Verbindungen der allgemeinen Formel I

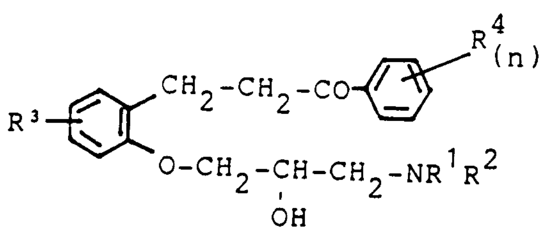

(I)

und ihre Säureadditionssalze sowie ein Verfahren zu ihrer Herstellung zur Verfügung zu stellen. Eine weitere Aufgabe ist es Arzneimittel, die eine Verbindung der allgemeinen Formel I oder deren Säureadditionssalz, vorzugsweise ein physiologisch verträgliches Säureadditionssalz, enthalten, zur Verfügung zu stellen.

In der allgemeinen Formel I sind $R^1$ und $R^2$ gleich oder verschieden und bedeuten Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu 9 C-Atomen, oder Phenylalkyl- oder Phenoxyalkylreste mit bis zu 6 C-Atomen im Alkylteil, wobei gegebenenfalls der Phenylrest durch einen Alkyl- oder Alkoxyrest mit jeweils bis zu 3 C-Atomen substituiert ist, oder
$R^1$ und $R^2$ bilden zusammen mit dem sie verbindenden Stickstoffatom einen 5- bis 7-gliedrigen gesättigten heterocyclischen Ring, der gegebenenfalls durch einen oder zwei Phenyl- und/oder Hydroxyreste substituiert sein und ein Sauerstoff- oder Stickstoffatomen als weiteres Heteroatom im Ring enthalten kann, wobei

das zusätzliche Stickstoffatom durch einen Alkylrest mit 1 bis 3 C-Atomen oder einen Phenylrest substituiert sein kann,
$R^3$ ist ein Wasserstoffatom, ein Alkylrest mit bis zu 3 C-Atomen, ein Fluor-, Chlor- oder Bromatom, eine Hydroxygruppe oder eine Alkoxygruppe mit bis zu 6 C-Atomen. $R^4$ ist ein Wasserstoffatom, ein Alkylrest mit bis zu 3 C-Atomen, ein Fluor-, Chlor- oder Bromatom, eine Hydroxygrupe oder eine Alkoxygruppe mit bis zu 6 C-Atomen und n ist eine ganze Zahl mit einem Wert von 1 bis 5.

Bevorzugt sind Verbindungen in denen $NR^1R^2$ eine tert.-Pentylamino-, n-Propylamino-, 1,1-Dimethylpropylamino-, Morphalino-, Isopropylamino-, tert.-Butylamino-, Pyrrolidino-, Piperidino- oder Cyclohexylaminogruppe ist;
der Rest $R^3$ ein Wasserstoffatom, eine Methyl-, Methoxy- oder Hydroxygruppe ist,
$R^4$ ein Wasserstoffatom, eine Methyl-, Methoxy- oder Hydroxygruppe ist und
n den Wert 1, 2 oder 3 hat.

Besonders bevorzugt sind Verbindungen, in denen $NR^1R^2$ eine n-Propylamino-, Isopropylamino-, tert.-Butylamino-, oder Piperidinogruppe ist,
der Rest $R^3$ ein Wasserstoffatom ist,
$R^4$ ein Wasserstoffatom oder eine Methoxygruppe ist und
n den Wert 1 oder 2 hat.

Am meisten bevorzugt sind:
1-(3,4-Dimethoxyphenyl)-3-[2'-(2-hydroxy-3-n-propylamino-propoxy)-phenyl]-1-propanon-hydrochlorid (Beispiel 1),
1-(3-Methoxyphenyl)-3-[2'-(2-hydroxy-3-isopropylaminopropoxy)-phenyl]-1-propanon-hydrochlorid (Beispiel 24),
1-Phenyl-3-[2'-(2-hydroxy-3-piperidinopropoxy)-phenyl]-1-propanon-hydrochlorid (Beispiel 47)
Die Erfindung betrifft ferner ein Verfahren zur Herstellung der Verbindungen der allgemeinen Formel I und ihrer Säureadditionssalze, das dadurch gekennzeichnet ist, daß man einen Phenoläther der allgemeinen Formel II

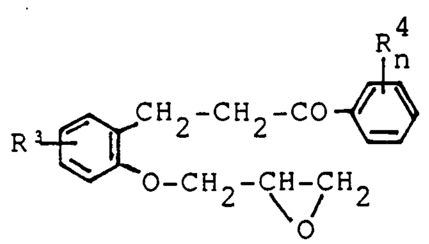

(II)

mit einem Amin der allgemeinen Formel III

$HNR^1R^2$ (III)

umsetzt.
$R^1$, $R^2$, $R^3$, $R^4$ und n haben die vorstehend angegebene Bedeutung.

Gegebenenfalls wird die erhaltene Verbindung der allgemeinen Formel I mit einer Säure in ein Säureadditionssalz überführt. Die Umsetzung kann beispielsweise nach dem in der EP-PS 0 074 014 beschriebenen Verfahren erfolgen.

Die Umsetzung wird bei Temperaturen von 10 bis 120 °C, d.h. bei Raumtemperatur oder bei höheren Temperaturen, zweckmäßig bei Temperaturen von 50 bis 120 °C, unter Atmosphärendruck oder in einem geschlossenen Gefäß unter erhöhtem Druck durchgeführt.

Die Ausgangsverbindungen der Formell II und III können ohne Verdünnungs- oder Lösungsmittel umgesetzt werden. Zweckmäßigerweise werden die Umsetzungen jedoch durchgeführt in Gegenwart eines inerten Verdünnungs- oder Lösungsmittels, bei spielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkytlglykoläthers oder cyclischen Äthers, wie Diäthyläther, 1,2-Dimethoxyäthan, Tetrahydrofuran oder Dioxan, eines Benzolkohlenwasserstoffs, wie Benzol selbst oder eines Alkylbenzols, insbesondere Toluol oder Xylol, oder eines aliphatischen Kohlenwasserstoffs, wie Hexan, Heptan oder Octan, Dimethylsulfoxid oder in Gegenwart von Wasser oder Mischungen der genannten Lösungsmittel.

Auch ist das in überschüssiger Menge verwendete Amin der allgemeinen Formel III gegebenenfalls als Verdünnungs- oder Lösungsmittel geeignet.

Die vollständige Umsetzung hängt von der Reaktionstemperatur ab und ist im allgemeinen innerhalb von 2 bis 15 Stunden beendet. Das Reaktionsprodukt kann in an sich bekannter Weise gewonnen werden, z.B. durch Filtration oder Abdestillieren des Verdünnungsmittels aus dem Reaktionsgemisch. Eine Reinigung der erhaltenen Verbindung erfolgt in üblicher Weise, beispielsweise durch Umkristallisation aus einem Lösungsmittel, Überführen in ein Säureadditionssalz oder durch Säulenchromatographie.

Der Phenoläther der allgemeinen Formel II kann durch Alkylierung eines 2'-Hydroxy-β-phenylpropiophenons der allgemeinen Formel IV

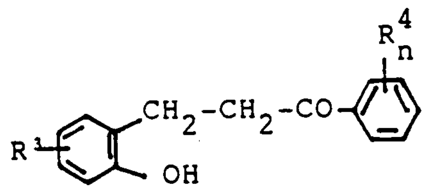

(IV)

mit einem Epihalogenhydrin erhalten werden. $R^3$, $R^4$ und n haben die vorstehend angegebene Bedeutung. Als Epihalogenhydrine kommen Epichlorhydrin, Epibromhydrin und Epijodhydrin in Betracht.

Die Umsetzung der Verbindungen IV zur Herstellung der Ausgangsverbindungen der allgemeinen Formel II wird zweckmäßig bei Temperaturen von 0 bis 120 °C und unter Normaldruck oder in einem geschlossenen Gefäß unter erhöhtem Druck durchgeführt. Als Lösungs- oder Verdünnungsmittel werden zweckmäßig ein niederes aliphatisches Keton, wie Aceton, Methyläthylketon oder Methylisobutylketon, ein niederer Alkohol mit 1 bis 4 C-Atomen, wie Methanol, Äthanol, Propanol oder Butanol, ein niederer aliphatischer oder cyclischer Äther, wie Diäthyläther, Tetrahydrofuran oder Dioxan, ein Dialkylformamid, wie Dimethylformamid oder Diäthylformamid, oder Dimethylsulfoxid oder Hexamethylphosphorsäuretriamid oder überschüssiges Alkylierungsmittel verwendet.

Bevorzugt werden die Umsetzungen in Gegenwart einer Base als säurebindendes Mittel durchgeführt. Geeignete Basen sind Alkalimetallcarbonate, -hydrogencarbonate, -hydroxide, -hydride oder -alkoholate, insbesondere von Natrium und Kalium, basische Oxide, wie Aluminiumoxid oder Calciumoxid, organische tertiäre Basen, wie Pyridin, niedere Trialkylamine, wie Trimethyl- oder Triäthylamin, oder Piperidin. Dabei können die Basen im Verhältnis zum eingesetzten Alkylierungsmittel in katalytischer Menge oder in stöchiometrischer Menge bzw. in geringem Überschuß verwendet werden.

Bevorzugt wird das 2'-Hydroxy-β-phenylpropionphenon mit Epichlorhydrin oder Epibromhydrin in einem polaren, aprotischen Lösungsmittel, insbesondere Dimethylsulfoxid, in Gegenwart von mindestens einem Moläquivalent Base, insbesondere Natriumhydrid, bezogen auf das Alkylierungsmittel, bei Temperaturen von 0 bis 50 °C umgesetzt.

Die Ausgangsverbindung der allgemeinen Formel IV, d.h. das 2-Hydroxy-β-phenylpropiophenon, und seine Herstellung ist bekannt.

Gegebenenfalls wird die erhaltene erfindungsgemäße Verbindung der allgemeinen Formel I in ein Säureadditionssalz, vorzugsweise in ein Salz einer physiologisch verträglichen Säure überführt. Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beschrieben z.B. in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977). Bevorzugt ist Salzsäure.

Die Säureadditionssalze werden in der Regel in an sich bekannter Weise durch Mischen der freien Base oder deren Lösungen mit der entsprechenden Säure oder deren Lösungen in einen organischen Lösungsmittel, beispielsweise einem niederen Alkohol, wie Methanol,Äthanol,n-Propanol oder Isopropanol, oder einem niederen Keton, wie Aceton, Methyläthylketon oder Methyl-isobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können physiologisch verträgliche wäßrige Lösungen von Säureadditionssalzen der Verbindung der Formel I in einer wäßrigen Säurelösung hergestellt werden.

Die Säureadditionssalze der Verbindungen der Formel allgemeinen I können in an sich bekannter Weise, z.B. mit Alkalien oder Ionenaustauschern, in die freie Base übergeführt werden. Von der freien Base lassen sich durch Umsetzung mit anorganischen oder organischen Säuren, insbesondere solchen, die zur

Bildung von therapeutisch verwendbaren Salzen geeignet sind, weitere Salze gewinnen. Diese oder auch andere Salze der neuen Verbindung, wie z.B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, intravenösen oder intramuskulären Applikation, die neben üblichen Träger- und Verdünnungsmitteln eine Verbindung der allgemeinen Formel I oder deren Säureadditionssalz als Wirkstoff enthalten, sowie die Verwendung der neuen Verbindung und ihrer physiologisch verträglichen Salze bei der Behandlung von Herzrhythmusstörungen.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmittel wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk,Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten, herstellen.

Die Einzeldosierung liegt beim Menschen
für die orale Anwendung zwischen 0,5 - 5 mg/kg
für die i.v. Anwwendung zwischen 0,05 - 2 mg/kg
für die i.m. Anwendung zwischen 0,1 - 3 mg/kg
für die rektale Anwendung zwischen 0,5 - 10 mg/kg.

Die erfindungsgemäßen Verbindungen und ihre physiologisch verträglichen Säureadditionssalze sind insbesondere wegen ihrer antiarrhythmischen und $\beta$-sympatholytischen Eigenschaften, vor allem zur Pharmakotherapie von Herzrhythmusstörungen, zur Behandlung der koronaren Herzkrankheit und zur Prophylaxe des plötzlichen Herztodes geeignet. Die antiarrhythmische Wirksamkeit der erfindungsgemäßen Verbindungen wurde sowohl anhand von elektrophysiologischen Studien an Purkinje-Fasern aus Hundeherzen sowie mit Hilfe von Ouabain-induzierten ventrikulären Tachykardien bei Hunden bestimmt.

Die erfindungsgemäßen Verbindungen werden in den nachfolgenden Tabellen (I) und (II) mit Propafenon (A) verglichen.

Die Wirkung auf die Kontraktionskraft des Herzens wurde am Meerschweinchen-Papillarmuskel untersucht. Hämodynamische Studien wurden bei gesunden, wie auch akut infarzierten Hunden durchgeführt. In der folgenden Tabelle (I) wird als wichtiges Kriterium der Sicherheitsfaktor (S.F.) dargestellt, der sich aus der Bezeihung zwischen der antiarrhythmischen Wirksamkeit, der negativen Inotropie und der spezifisch stärkeren Wirksamkeit bei höheren Frequenzen nach der Formel:

$$S.F. = \text{Rate Faktor von } V_{max} \cdot (C20CF/C20V_{max})$$

ergibt. Vergleichend sie hierbei erwähnt, daß die zur Zeit führenden Antiarrhythmika Propafenon und

Flecainid einen S.F. von 1,5 bzw. 1,7 haben.

Als zusätzliches Kriterium wurde der Vorzeitigkeitsindex (Prematurity-Faktor) bestimmt, der die erwünschte Wirksamkeit der erfindungsgemäßen Verbindungen bei vorzeitigen Extrasystolen charakterisiert.

Die hohe antiarrhythmische Wirksamkeit ist nicht begleitet von einer signifikanten Toxizitätssteigerung im Vergleich zu Propafenon. Dies ergibt sich aus einem Vergleich der $LD_{50}$-Werte bei Ratten und Mäusen, die in Tabelle (II) zusammengefaßt sind.

Tabelle I

| Testverbindung | C20 CF (μM) | C20 Vmax (μM) | Rate-Faktor von Vmax | Prematurity Faktor Vmax | S.-F. |
|---|---|---|---|---|---|
| 1 | 15,0 | 12,5 | 5,00 | 0,50 | 6,0 |
| 2 | 8,0 | 3,6 | 1,27 | 1,15 | 2,8 |
| 4 | 22,0 | 4,8 | 1,13 | 1,01 | 5,2 |
| 5 | 12,0 | 4,4 | 1,39 | 0,98 | 3,8 |
| 6 | 20,0 | 11,3 | 1,32 | 0,70 | 2,3 |
| 7 | 18,0 | 5,7 | 1,39 | 0,98 | 4,4 |
| 8 | 13,0 | 8,3 | 1,34 | 0,97 | 2,1 |
| 9 | 25,0 | 10,2 | 1,30 | 0,97 | 3,2 |
| 12 | 18,0 | 5,8 | 1,14 | 0,90 | 3,5 |
| 16 | 9,0 | 11,9 | 5,00 | 0,50 | 3,8 |
| 24 | 10,0 | 1,6 | 1,15 | 1,01 | 7,2 |
| 26 | 3,0 | 1,7 | 1,16 | 0,93 | 2,1 |
| 27 | 4,0 | 1,6 | 0,99 | 1,24 | 2,5 |
| 38 | 7,0 | 1,7 | 1,30 | 0,97 | 5,4 |
| 44 | 6,0 | 2,6 | 1,11 | 0,99 | 2,6 |
| 46 | 2,0 | 2,2 | 2,50 | 0,92 | 2,3 |
| 47 | 2,0 | 0,3 | 1,08 | 0,99 | 7,2 |
| 48 | 3,0 | 1,0 | 1,18 | 0,91 | 3,5 |
| A | 5,3 | 3,7 | 1,08 | 0,97 | 1,5 |

C20 CF: konzentration, die die Kontraktion des Papillarmuskels um 20 % verringert

C20 Vmax: Konzentration, die Vmax bei einer Zykluslänge von 1000 mSek. um 20 % verringert

Rate-Faktor von Vmax: (% des Kontrollwertes Vmax bei einer Zykluslänge von 2000) / (% des Kontrollwertes Vmax bei einer Zykluslänge von 500) bei C20 Vmax

Prematurity Faktor Vmax: (% des Kontrollwertes Vmax bei vorzeitigen Extrasystolen) / (% des Kontrollwertes Vmax bei einem Normalschlag) bei Vmax

S.F.: Sicherheitsfaktor, errechnet als Rate-Faktor von Vmax * (C20 CF / C20 Vmax)

Tabelle II

$LD_{50}$ (mg/kg)

| Test-verbindung | Ratte (i.v.) | Maus (i.v.) |
|---|---|---|
| A | 16 | 18 |
| Beisp. 1 | 17 | 16 |
| " 38 | 15 | 17 |

Die Beispiele erläutern die Erfindung.

A) Herstellung der Ausgangsverbindungen

Beispiel I
1-(3,4-Dimethoxyphenyl)-3-[2'-(1,2-epoxy-3-propoxy)-phenyl]-1-propanon

275,4 g (0,96 Mol) 3,4-Dimethoxy-β-2'-hydroxyphenyl-propiophenon werden mit 600 ml Epichlorhydrin, 300 ml 2-Propanol und 38,5 g (0,97 Mol) Natriumhydroxid 5 h unter Rückfluß erhitzt und gerührt. Das entstandene Salz wird abfiltriert und die Lösung unter vermindertem Druck zur Trockene eingeengt. Der ölige Rückstand wird ohne Reinigung in die nächste Stufe eingesetzt. Ausbeute: 334,2 g( 100 %) der Titelverbindung.

Analog wurden folgende Verbindungen hergestellt:
1-(3,4,5-Trimethoxyphenyl)-3-[2'-(1,2-epoxy-3-propoxy)-phenyl]-1-propanon
1-(3,4,5-Trimethylphenyl)-3-[2'-(1,2-epoxy-3-propoxy)-phenyl]-1-propanon
1-(2,4,6-Trimethylphenyl)-3-[2'-(1,2-epoxy-3-propoxy)-phenyl]-1-propanon
1-Phenyl-3-[2'-(1,2-epoxy-3-propoxy)-4'-methoxyphenyl]-1-propanon
1-(3-Methoxyphenyl)-3-[2'-(1,2-epoxy-3-propoxy)-phenyl]-1-propanon
1-(4-Methoxyphenyl)-3-[2'-(1,2-epoxy-3-propoxy)-phenyl]-1-propanon
1-(2-Methoxyphenyl)-3-[2'-(1,2-epoxy-3-propoxy)-phenyl]-1-propanon
1-(4-Methylphenyl)-3-[2'-(1,2-epoxy-3-propoxy)-phenyl]-1-propanon
1-Phenyl-3-[2'-(1,2-epoxy-3-propoxy)-phenyl]-1-propanon

B) Herstellung der erfindungsgemäßen Verbindungen

Beispiel 1
1-(3,4-Dimethoxyphenyl)-3-[2'-(2-hydroxy-3-n-propylaminopropoxy)-phenyl]-1-propanon-hydrochlorid

11,3 g (0,033 Mol) 1-(3,4-Dimethoxyphenyl)-3-[2'-(1,2-epoxy-3-propoxy)-phenyl]-1-propanon und 5,9 g (0,099 Mol) n-Propylamin werden in 100 ml Methanol gelöst und 3 1/2 h unter Rückfluß erhitzt. Das Lösemittel und das überschüssige Amin werden unter vermindertem Druck entfernt. Der ölige Rückstand wird in 150 ml Isopropanol gelöst und mit konzentrierter Salzsäure versetzt. Nach dem Erhitzen und Kühlen der Lösung werden Kristalle erhalten, die abgesaugt, getrocknet und aus Isopropanol umkristallisiert werden. Ausbeute 8,2 g (56 %) Der Titelverbindung vom Fp. 126 °C.

Analog wurden hergestellt (Beispiele 2 bis 49):

2. 1-(3,4-Dimethoxyphenyl)-3-[2'-(2-hydroxy-3-tert.-pentylamino-propoxy)-phenyl]-1-propanon-hydrochlorid, Fp.: 151-152 °C.
3. 1-(3,4-Dimethoxyphenyl)-3-[2'-(2-hydroxy-3-morpholino-propoxy)-phenyl]-1-propanon-hydrochlorid, Fp.: 150-151 °C.
4. 1-(3,4-Dimethoxyphenyl)-3-[2'-(2-hydroxy-3-isopropylamino-propoxy)-phenyl]-1-propanon-hydrochlorid, Fp.: 135-136 °C.
5. 1-(3,4-Dimethoxyphenyl)-3-[2'-(2-hydroxy-3-tert.-butylamino-propoxy)-phenyl]-1-propanon-hydrochlorid, Fp.:L 164-165 °C.
6.1-(3,4-Dimethoxyphenyl)-3-[2'-hydroxy-3-piperidino-propoxy)-phenyl]-1-propanon-hydrochlorid, Fp.: 124-125 °C.
7. 1-(3,4-Dimethoxyphenyl)-3-[2'-(2-hydroxy-3(2''-hydroxy-propylamino)-propoxy)-phenyl]-1-propanon-hydrochlorid, Fp.: 109-111 °C.
8. 1-(3,4-Dimethoxyphenyl)-3-[2'-(2-hydroxy-3(2''-hydroxyethylamino)-propoxy)-phenyl]-1-propanon-hydrochlorid, Fp.: 146-147,5 °C.
9. 1-(3,4-Dimethoxyphenyl)-3-[2'-(2-hydroxy-3-(1''-hydroxybut-2-ylamino)-propoxy)-phenyl]-1-propanon-oxalat, Fp.: 195-197 °C.
10. 1-(3,4,5-Trimethoxyphenyl)-3-[2'-(2-hydroxy-3-tert.-pentylamino-propoxy)-phenyl]-1-propanon-hydrochlorid, Fp.: 179-180 °C.
11. 1-(3,4,5-Trimethoxyphenyl)-3-[2'-(hydroxy-3-n-propylamino-propoxy)-phenyl]-1-propanon-hydrochlorid, Fp.: 140-142 °C.
12. 1-(3,4,5-Trimethoxyphenyl)-3-[2'-(2-hydroxy-3-isopropylamino-propoxy)-phenyl]-1-propanon-hydrochlorid, Fp.: 161-162 °C.
13. 1-(3,4,5-Trimethoxyphenyl)-3-[2'-(2-hydroxy-3-tert.-butylamino-propoxy)-phenyl]-1-propanon-hydrochlorid, Fp.: 178,5-179,5 °C.

14. 1-(3,4,5-Trimethoxyphenyl)-3-[2′-(2-hydroxy-3-piperidino-propoxy)-phenyl]-1-propanon-hydrochlorid, Fp.: 148,5-150 °C.

15. 1-(3,4,5-Trimethoxyphenyl)-3-[2′-(2-hydroxy-3-morpholino-propoxy)-phenyl]-1-propanon-hydrochlorid, Fp.: 166,5-167,5 °C.

16. 1-(3,4,5-Trimethoxyphenyl)-3-[2′-(2-hydroxy-3-cyclohexylamino-propoxy)-phenyl]-1-propanon-hydrochlorid, Fp.: 158,5-159,5 °C.

17. 1-(3,4,5-Trimethylphenyl)-3-[2′-(2-hydroxy-3-piperidino-propoxy)-phenyl]-1-propanon-hydrochlorid, Fp.: 133-134 °C.

18. 1-(2,4,6-Trimethylphenyl)-3-[2′-(2-hydroxy-3-n-propylamino-propoxy)-phenyl]-1-propanon-oxalat, Fp.: 184-185 °C.

19. 1-(2,4,6-Trimethylphenyl)-3-(2′-(2-hydroxy-3-tert.-butylamino-propoxy)-phenyl]-1-propanon-oxalat, Fp.: 148-150 °C.

20. 1-(2,4,6-Trimethylphenyl)-3-[2′-(2-hydroxy-3-tert.-pentylamino-propoxy)-phenyl]-1-propanon-hydrochlorid, Fp.: 118-120 °C.

21. 1-(2,4,6-Trimethylphenyl)-3-[2′-(2-hydroxy-3-isopropylamino-propoxy)-phenyl]-1-propanon-hydrochlorid. Fp.: 111-112 °C.

22. 1-(2,4,6-Trimethylphenyl)-3-[2′-(2-hydroxy-3-morpholino-propoxy)-phenyl]-1-propanon-hydrochlorid, Fp.: 132-133 °C.

23. 1-Phenyl-3-[2′-(2-hydroxy-3-tert.-pentylaminopropoxy)-4′-methoxyphenyl]-1-propanon-oxalat, Fp.:118-120 °C.

24. 1-(3-Methoxyphenyl)-3-[2′-(2-hydroxy-3-isopropylamino-propoxy)-phenyl]-1-propanon-hydrochlorid, Fp.: 109,5 °C.

25. 1-(3-Methoxyphenyl)-3-[2′-(2-hydroxy-3-tert.-pentylamino-propoxy)-phenyl]-1-propanon-hydrochlorid, Fp.: 113,5 °C.

26. 1-(3-Methoxyphenyl)-3-[2′-(2-hydroxy-3-n-propylamino-propoxy)-phenyl]-1-propanon-hydrochlorid, Fp.: 105,5 °C.

27. 1-(3-Methoxyphenyl)-3-[2′-(2-hydroxy-3-tert.-butylamino-propoxy)-phenyl]-1-propanon-hydrochlorid, Fp.: 110-111 °C.

28. 1-(3-Methoxyphenyl)-3-[2′-(2-hydroxy-3-morpholino-propoxy)-phenyl]-1-propanon-hydrochlorid, Fp.: 124,5 °C.

29.1-(4-Methoxyphenyl)-3-[2′-(2-hydroxy-3-tert.-pentylamino-propoxy)-phenyl]-1-propanon-hydrochlorid, Fp.: 114,5-115,5 °C.

30. 1-(4-Methoxyphenyl)-3-[2′-(2-hydroxy-3-piperidino-propoxy)-phenyl]-1-propanon-hydrochlorid, Fp.: 151 °C.

31. 1-(4-Methoxyphenyl)-3-[2′-(2-hydroxy-3-n-propylamino-propoxy)-phenyl]-1-propanon-hydrochlorid, Fp.: 115,5-116,5 °C.

32. 1-(4-Methoxyphenyl)-3-[2′-(2-hydroxy-3-tert.-butylamino-propoxy)-phenyl]-1-propanon-hydrochlorid, Fp.: 142-143 °C.

33. 1-(2-Methoxyphenyl)-3-[2′-(2-hydroxy-3-isopropylamino-propoxy)-phenyl]-1-propanon-hydrochlorid, Fp.: 147-148 °C.

34. 1-(2-Methoxyphenyl)-3-[2′-(2-hydroxy-3-tert.-pentylamino-propoxy)-phenyl]-1-propanon-hydrochlorid, Fp.: 146-149 °C.

35. 1-(2-Methoxyphenyl)-3-[2′-(2-hydroxy-3-piperidino-propoxy)-phenyl]-1-propanon-hydrochlorid, Fp.: 107,5-109,5 °C.

36. 1-(2-Methoxyphenyl)-3-[2′-(2-hydroxy-3-(4″-methyl-1″-piperazinyl-propoxy)-phenyl]-1-propanon-hydrochlorid, Fp.: 172-175 °C.

37. 1-(2-Methoxyphenyl)-3-[2′-(2-hydroxy-3-n-propylamino-propoxy)-phenyl]-1-propanon-hydrochlorid, Fp.: 125-128 °C.

38. 1-(2-Methoxyphenyl)-3-[2′-(2-hydroxy-3-tert.-butylamino-propoxy)-phenyl]-1-propanon-hydrochlorid, Fp.: 145-147 °C.

39. 1-(2-Methoxyphenyl)-3-[2′-(2-hydroxy-3-morpholino-propoxy)-phenyl]-1-propanon-hydrochlorid, Fp.: 94-96 °C.

40. 1-(4-Methylphenyl)-3-[2′-(2-hydroxy-3-isopropylamino-propoxy)-phenyl]-1-propanon-hydrochlorid, Fp.: 126,5-128,5 °C.

41. 1-(4-Methylphenyl)-3-[2′-(2-hydroxy-3-cyclohexylamino-propoxy)-phenyl]-1-propanon-hydrochlorid, Fp.: 154-156 °C.

42. 1-(4-Methylphenyl)-3-[2′-(2-hydroxy-3-tert.-pentylamino-propoxy)-phenyl]-1-propanon-hydrochlorid,

Fp.: 120,5-121,5 °C.

43. 1-(4-Methylphenyl)-3-[2′-(2-hydroxy-3-n-propylamino-propoxy)-phenyl]-1-propanon-hydrochlorid, Fp.: 109-111 °C.

44. 1-(4-Methylphenyl)-3-[2′-(2-hydroxy-3-tert.-butylamino-propoxy)-phenyl]-1-propanon-hydrochlorid, Fp.: 132,5-133,5 °C.

45. 1-Phenyl-3-[2′-(2-hydroxy-3-isopropylamino-propoxy)-phenyl]-1-propanon-hydrochlorid, Fp.: 85-90 °C.

46. 1-Phenyl-3-[2′-(2-hydroxy-3-cyclohexylamino-propoxy)-phenyl]-1-propanon-hydrochlorid, Fp.: 137-140 °C.

47. 1-Phenyl-3-[2′-(2-hydroxy-3-piperidino-propoxy)-phenyl]-1-propanon-hydrochlorid, Fp.: 132-133 °C.

48. 1-Phenyl-3-[2′-(2-hydroxy-3-tert.-butylamino-propoxy)-phenyl]-1-propanon-hydrochlorid, Fp.: 108-109 °C.

49. 1-Phenyl-3-[2′-(2-hydroxy-3-(3″-methoxypropylamino)-propoxy)-phenyl]-1-propanon-hydrochlorid, Fp.: 114-116 °C.

**Beispiel 50**

Herstellungsvorschrift für Tabletten

a) Rezeptur

| | |
|---|---|
| Verbindung von Beispiel 1 | 75,00 g |
| Mikrokristalline Cellulose (Pulver, 50 µm) | 15,75 g |
| Poly-(1-vinyl-2-pyrrolidon) | 5,00 g |
| Hydroxypropylmethylcellulose 2910 | 3,75 g |
| Magnesiumstearat | 0,50 g |
| | 100,00 g |

b) Mischen und Granulieren

Die Verbindung von Beispiel 1 wird bei Bedarf gesiebt, dann werden alle Rohstoffe außer Magnesiumstearat in einem Mischer gemischt und ebenfalls im Mischer mit einer geeigneten Menge einer Granulierflüssigkeit (z.B. Wasser oder Isopropanol-Dichlormethan 1:1) befeuchtet. Die feuchte Mischung wird mit einem geeigneten Sieb gesiebt, im Trockenschrank getrocknet und nochmals gesiebt. Das trockene Granulat wird mit dem Magnesiumstearat im Mischer vermengt.

c) Pressen von Tabletten

Aus der nach a) hergestellten Mischung werden in einer Tablettenpresse Tabletten mit einem Gewicht zwischen 40 und 400 mg gepreßt. Die Preßkraft und der Tablettendurchmesser werden so gewählt, daß Zerfallzeit im Testgerät nach Ph. Eur. unter 15 Minuten liegt und die Tabletten mechanisch genügend stabil sind.

**Beispiel 51**

Herstellungsvorschrift für Filmtabletten

A. Rezeptur

a) Tablette
(siehe Herstellungsvorschrift für Tabletten, Beispiel 50)
b) Filmüberzug
Gesamtauftragsmenge 5-20 % des Tablettengewichts davon:
Hydroxypropylmethylcellulose 2910 77 %
Macrogol® 6000(Weichmacher) 23 %

B. Herstellung der Tabletten
(siehe Herstellungsvorschrift für Tabletten, Beispiel 50)

C. Herstellung der Filmtabletten

Die Bestandteile des Filmüberzugs werden in einem geeigneten Lösungsmittel (z.B. Wasser oder Äthanol/Wasser 70:30) gelöst. Die Tabletten werden in einer Filmcoating-Anlage mit der Lösung des Filmbildners und des Weichmachers besprüht und im Heißluftstrom getrocknet. Die Filmtabletten werden im Trockenschrank nachgetrocknet.

**Beispiel 52**

A. Rezeptur

a) Drageekern
(siehe Herstellungsvorschrift für Tabletten)
b) Drageedecke
Gesamtauftragsmenge 25-100 % des Kerngewichts davon:

| | |
|---|---|
| Saccharose | 51,4 % |
| Talkum | 24,0 % |
| Calciumsulfat-Hemihydrat | 10,3 % |
| Stärkesirup | 5,0 % |
| Arabisches Gummi | 3,9 % |
| Macrogol® 6000 | 2,9 % |
| Titan(IV)-oxid | 1,6 % |
| Hochdisperses Siliciumdioxid | 0,8 % |
| Natriumdodecylsulfat | 0,1 % |
| | 100,0 % |

B. Herstellung der Drageekerne
(siehe Herstellungsvorschrift für Tabletten)

C. Herstellung der Dragees

Zusammensetzung der verwendeten Dragierlösung, Andeckmischung und Dragiersuspension

a) Dragierlösung

| | |
|---|---|
| Saccharose | 47,6 % |
| Stärkesirup | 19,1 % |
| Arabisches Gummi | 3,8 % |
| dest. Wasser | 29,5 % |
| | 100,0 % |

b) Andeckmischung

| | |
|---|---|
| Talkum<br>Calciumsulfat-Hemihydrat<br>Hochdisperses Siliciumdioxid | 70,0 %<br>26,7 %<br>3,3 %<br>100,0 % |

c) Dragiersuspension

| | |
|---|---|
| Saccharose<br>Talkum<br>Calciumsulfat-Hemihydrat<br>Arabisches Gummi<br>Stärkesirup<br>Macrogol® 6000<br>Titan (IV)-oxid<br>Natriumdodecylsulfat<br>dest. Wasser | 47,8 %<br>9,6 %<br>4,8 %<br>3,6 %<br>3,2 %<br>2,9 %<br>1,6 %<br>0,1 %<br>26,4 %<br>100,0 % |

Überziehen der Drageekerne

Die Drageekerne werden zunächst im rotierenden Kessel mit der Dragierlösung angefeuchtet und dann mit soviel Andeckmischung abgepudert bis sie wieder frei rollen. Nach dem Trocknen der Kerne wird dieser Vorgang wiederholt. Dann werden die Kerne im Trockenschrank nachgetrocknet. Danach werden die Kerne mit der Dragiersuspension schichtweise überzogen, bis das gewünschte Endgewicht erreicht ist. Jeweils nach dem Auftrag einer Schicht müssen die Kerne getrocknet werden.

**Beispiel 53**

Herstellungsvorschrift für Kapseln

A. Wirkstoffdosierung ab 75 mg

1. Rezeptur

| | |
|---|---|
| Verbindung von Beispiel 2<br>Mikrokristalline Cellulose Pulver, 50 µm<br>Poly-(1-vinyl-2-pyrrolidon)<br>Hydroxypropylmethylcellulose 2910 | 75,00 g<br>16,25 g<br>5,00 g<br>3,75 g<br>100,00 g |

2. Mischen und Granulieren gemäß Beispiel 50b)

3. Abfüllen des Granulats in Kapseln

Das Granulat wird mit Hilfe einer Kapselfüllmaschine in Hartgelatinekapseln der Größe 3, 2, 1 oder 0 abgefüllt. Die Füllmenge pro Kapsel richtet sich dabei nach der gewünschten Dosierung des Wirkstoffes.

B. Wirkstoffdosierung 30-75 mg

1. Rezeptur

| | |
|---|---|
| Verbindung von Beispiel 4 | 30,00 - 75,00 g |
| Mikrokristalline Cellulose Pulver, 50 µm | 61,25 - 16,25 g |
| Poly-(1-vinyl-2-pyrrolidon) | 5,00 g |
| Hydroxypropylmethylcellulose 2910 | 3,75 g |
| | 100,00 g |

Die Summe der Einwaagen von Wirkstoff und mikrokristalliner Cellulose soll immer 91,25 g betragen. Die Wirkstoffmenge beträgt das Tausendfache der Einzeldosierung.

2. Mischen und Granulieren gemäß Beispiel 50b)

3. Abfüllen des Granulats in Kapseln

Je 100 mg Granulat werden mit Hilfe einer Kapselfüllmaschine in Hartgelatinekapseln der Größe 3 oder 2 abgefüllt.

**Beispiel 54**

Herstellungsvorschrift für Ampullen

1. Rezeptur

| | |
|---|---|
| Verbindung von Beispiel 8 | 1,5g |
| Wasser für Injektionszwecke ad | 100,0 ml |

2. Herstellung der Lösung

90% des für die gewählte Ansatzgröße benötigten Wassers werden vorgelegt; der Wirkstoff wird unter Erwärmen darin gelöst. Die Lösung wird nach dem Abkühlen auf das Endvolumen aufgefüllt.

3. Abfüllen der Lösung

Die fertige Lösung wird in Glasampullen, deren Füllmenge sich nach der gewünschten Dosierung richtet, abgefüllt. Sodann werden die Glasampullen zugeschmolzen.

4. Sterilisation

Die Ampullen werden 20 Minuten bei 120 °C im Dampf sterilisiert.

**Beispiel 55**

Herstellungsvorschrift für Suppositorien

a. Rezeptur

| | |
|---|---|
| Verbindung von Beispiel 22 | 30 - 200 mg |
| Hartfett (Schmelzpunkt 35-36,5 °C) ad | 2000 mg |

b. Herstellung der wirkstoffhaltigen Schmelze

Die für eine bestimmte Anzahl Suppositorien benötigte Menge Hartfett wird bei 40 °C im Wasserbad geschmolzen. Der Wirkstoff wird durch ein 0,8 mm Sieb gedrückt und in die Schmelze eingerührt, so daß eine Suspension entsteht.

c. Herstellung der Suppositorien

Die Schmelze wird auf 37-38 °C abkühlen gelassen und unter ständigem Rühren in Zäpfchenformen so

abgefüllt, daß das Gewicht eines Suppositoriums 2000 mg beträgt. Die Zäpfchenform wird nach dem Erstarren der Schmelze verschlossen.

**Patentansprüche**

**Patentansprüche für folgende Vertragsstaaten : AT, BE, CH, DE, FR, GB, IT, LI, LU, NL, SE**

**1.** Aminopropanolderivate von 3-(2'-Hydroxyphenyl)-1-propanon-Verbindungen der allgemeinen Formel I

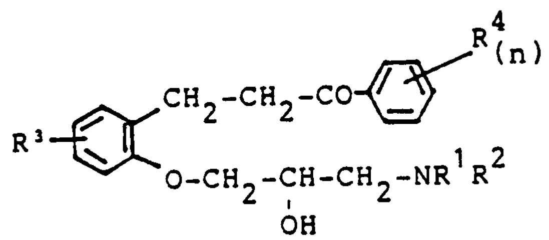

(I)

in der $R^1$ und $R^2$ gleich oder verschieden sind und Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu 9 C-Atomen oder Phenylalkyl- oder Phenoxyalkylreste mit bis zu 6 C-Atomen im Alkylteil und gegebenenfalls der Phenylrest durch einen Alkyl- oder Alkoxyrest mit jeweils bis zu 3 C-Atomen substituiert ist, bedeuten oder
$R^1$ und $R^2$ zusammen mit dem sie verbindenden Stickstoffatom einen 5- bis 7-gliedrigen gesättigten heterocyclischen Ring bilden, der gegebenenfalls durch einen oder zwei Phenyl- und/oder Hydroxyreste substituiert sein und ein Sauerstoffatom oder Stickstoffatom als weiteres Heteroatom im Ring enthalten kann, wobei das zusätzliche Stickstoffatom durch einen Alkylrest mit 1 bis 3 C-Atomen oder einen Phenylrest substituiert sein kann,
$R^3$ ein Wasserstoffatom, ein Alkylrest mit bis zu 3 C-Atomen, ein Fluor-, Chlor- oder Bromatom, eine Hydroxygruppe, eine Alkoxygruppe mit bis zu 6 C-Atomen ist,
$R^4$ ein Wasserstoffatom, ein Alkylrest mit bis zu 3 C-Atomen, ein Fluor-, Chlor- oder Bromatom, eine Hydroxygruppe, oder eine Alkoxygruppe mit bis zu 6 C-Atomen ist, und
n eine ganze Zahl mit einem Wert von 1 bis 5 ist, und ihre Säureadditionssalze.

**2.** Verbindungen nach Anspruch 1, dadurch gekennzeichnet, daß $NR^1R^2$ eine tert.-Pentylamino-, n-Propylamino-, 1,1-Dimethylpropylamino-, Morpholino-, Isopropylamino-, tert.-Butylamino-, Pyrrolidino-, Piperidino- oder Cyclohexylaminogruppe ist, der Rest $R^3$ ein Wasserstoffatom, eine Methyl-, Methoxy- oder Hydroxygruppe ist,
$R^4$ ein Wasserstoffatom, eine Methyl-, Methoxy- oder Hydroxygruppe ist, und n den Wert 1, 2 oder 3 hat.

**3.** Verbindungen nach Anspruch 1, dadurch gekennzeichnet, daß $NR^1R^2$ eine n-Propylamino-, Isopropylamino-, tert.-Butylamino-, oder Piperidinogruppe ist,
der Rest $R^3$ ein Wasserstoffatom ist,
$R^4$ ein Wasserstoffatom oder eine Methoxygruppe ist, und n den Wert 1 oder 2 hat.

**4.** 1-(3,4-Dimethoxyphenyl)-3-[2'-(hydroxy-3-n-propylamino-propoxy)-phenyl]-1-propanon-hydrochlorid.

**5.** 1-(3-Methoxyphenyl)-3-[2'-(2-hydroxy-3-isopropylamino-propoxy)-phenyl]-1-propanon-hydrochlorid.

**6.** 1-Phenyl-3-[2'-(2-hydroxy-3-piperidino-propoxy)-phenyl]-1-propanon-hydrochlorid.

**7.** Verfahren zur Herstellung der Verbindungen nach Anspruch 1 und ihrer Säureadditionssalze, dadurch gekennzeichnet, daß man einen Phenoläther der allgemeinen Formel II

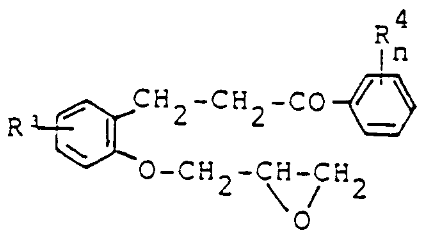

(II)

in der $R^3$, $R^4$ und n die in Anspruch 1 angegebene Bedeutung haben, mit einem Amin der allgemeinen Formel III

$HNR^1R^2$ (III)

in der $R^1$ und $R^2$ die in Anspruch 1 bis 6 angegebene Bedeutung haben, umsetzt und gegebenenfalls die erhaltene Verbindung mit einer Säure in ein Säureadditionssalz überführt.

**8.** Arzneimittel, gekennzeichnet durch einen Gehalt an einer Verbindung gemäß einem der Ansprüche 1 bis 6.

**Patentansprüche für folgenden Vertragsstaat : ES**

**1.** Verfahren zur Herstellung von Aminopropanolderivaten von 3-(2'-Hydroxyphenyl)-1-propanon-Verbindungen der allgemeinen Formel I

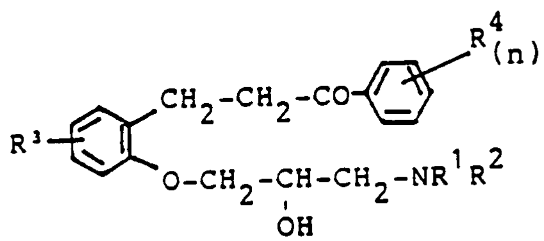

(I)

in der $R^1$ und $R^2$ gleich oder verschieden sind und Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu 9 C-Atomen oder Phenylalkyl- oder Phenoxyalkylreste mit bis zu 6 C-Atomen im Alkylteil und gegebenenfalls der Phenylrest durch einen Alkyl- oder Alkoxyrest mit jeweils bis zu 3 C-Atomen substituiert ist, bedeuten oder
$R^1$ und $R^2$ zusammen mit dem sie verbindenden Stickstoffatom einen 5- bis 7-gliedrigen gesättigten heterocyclischen Ring bilden, der gegebenenfalls durch einen oder zwei Phenyl- und/oder Hydroxyreste substituiert sein und ein Sauerstoffatom oder Stickstoffatom als weiteres Heteroatom im Ring enthalten kann, wobei das zusätzliche Stickstoffatom durch einen Alkylrest mit 1 bis 3 C-Atomen oder einen Phenylrest substituiert sein kann,
$R^3$ ein Wasserstoffatom, ein Alkylrest mit bis zu 3 C-Atomen, ein Fluor-, Chlor- oder Bromatom, eine Hydroxygruppe, eine Alkoxygruppe mit bis zu 6 C-Atomen ist,
$R^4$ ein Wasserstoffatom, ein Alkylrest mit bis zu 3 C-Atomen, ein Fluor-, Chlor- oder Bromatom, eine Hydroxygruppe, oder eine Alkoxygruppe mit bis zu 6 C-Atomen ist, und
n eine ganze Zahl mit einem Wert von 1 bis 5 ist, und ihren Säureadditionssalzen, dadurch gekennzeichnet, daß man einen Phenoläther der allgemeinen Formel II

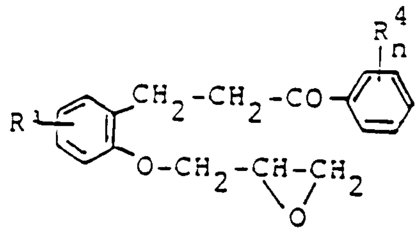

(II)

in der $R^3$, $R^4$ und n die vorstehende Bedeutung haben, mit einem Amin der allgemeinen Formel III

$HNR^1R^2$ (III)

in der $R^1$ und $R^2$ die vorstehende Bedeutung haben, umsetzt und gegebenenfalls die erhaltene Verbindung mit einer Säure in ein Säureadditionssalz überführt.

**2.** Verfahren nach Anspruch 1, dadurch gekennzeichnet, daß $NR^1R^2$ eine tert.-Pentylamino-, n-Propylamino-, 1,1-Dimethylpropylamino-, Morpholino-, Isopropylamino-, tert.-Butylamino-, Pyrrolidino-, Piperidino- oder Cyclohexylaminogruppe ist, der Rest $R^3$ ein Wasserstoffatom, eine Methyl-, Methoxy- oder Hydroxygruppe ist ,
$R^4$ ein Wasserstoffatom, eine Methyl-, Methoxy- oder Hydroxygruppe ist, und n den Wert 1, 2 oder 3 hat.

**3.** Verfahren nach Anspruch 1, dadurch gekennzeichnet, daß $NR^1R^2$ eine n-Propylamino-, Isopropylamino-, tert.-Butylamino-, oder Piperidinogruppe ist,
der Rest $R^3$ ein Wasserstoffatom ist,
$R^4$ ein Wasserstoffatom oder eine Methoxygruppe ist, und
n den Wert 1 oder 2 hat.

**4.** Verfahren nach Anspruch 1 zur Herstellung von 1-(3,4-Dimethoxyphenyl)-3-[2'-(2-hydroxy-3-n-propylamino-propoxy)-phenyl]-1-propanon-hydrochlorid.

**5.** Verfahren nach Anspruch 1 zur Herstellung von 1-(3-Methoxyphenyl)-3-[2'-(2-hydroxy-3-isopropylamino-propoxy)-phenyl]-1-propanon-hydrochlorid.

**6.** Verfahren nach Anspruch 1 zur Herstellung von 1-Phenyl-3-[2'-(2-hydroxy-3-piperidino-propoxy)-phenyl]-1-propanon-hydrochlorid.

**Patentansprüche für folgenden Vertragsstaat : GR**

**1.** Aminopropanolderivate von 3-(2'-Hydroxyphenyl)-1-propanon-Verbindungen der allgemeinen Formel I

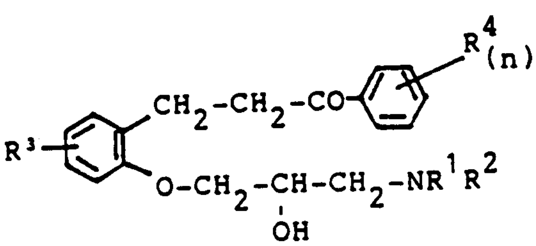

(I)

in der $R^1$ und $R^2$ gleich oder verschieden sind und Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu 9 C-Atomen oder Phenylalkyl- oder Phenoxyalkylreste mit bis zu 6 C-Atomen im Alkylteil und gegebenenfalls der Phenylrest durch einen Alkyl- oder Alkoxyrest mit jeweils bis zu 3 C-Atomen substituiert ist, bedeuten oder
$R^1$ und $R^2$ zusammen mit dem sie verbindenden Stickstoffatom einen 5- bis 7-gliedrigen gesättigten heterocyclischen Ring bilden, der gegebenenfalls durch einen oder zwei Phenyl- und/oder Hydroxyreste substituiert sein und ein Sauerstoffatom oder Stickstoffatom als weiteres Heteroatom im Ring enthalten kann, wobei das zusätzliche Stickstoffatom durch einen Alkylrest mit 1 bis 3 C-Atomen oder einen Phenylrest substituiert sein kann,
$R^3$ ein Wasserstoffatom, ein Alkylrest mit bis zu 3 C-Atomen, ein Fluor-, Chlor- oder Bromatom, eine Hydroxygruppe, eine Alkoxygruppe mit bis zu 6 C-Atomen ist,
$R^4$ ein Wasserstoffatom, ein Alkylrest mit bis zu 3 C-Atomen, ein Fluor-, Chlor- oder Bromatom, eine Hydroxygruppe, oder eine Alkoxygruppe mit bis zu 6 C-Atomen ist, und
n eine ganze Zahl mit einem Wert von 1 bis 5 ist, und ihre Säureadditionssalze.

**2.** Verbindungen nach Anspruch 1, dadurch gekennzeichnet, daß $NR^1R^2$ eine tert.-Pentylamino, n-Propylamino, 1,1-Dimethylpropylamino-,Morpholino-, Isopropylamino-, tert.-Butylamino-, Pyrrolidino-, Piperidino- oder Cyclohexylaminogruppe ist, der Rest $R^3$ ein Wasserstoffatom, eine Methyl-, Methoxy- oder Hydroxygruppe ist ,
$R^4$ ein Wasserstoffatom, eine Methyl-, Methoxy- oder Hydroxygruppe ist, und n den Wert 1, 2 oder 3 hat.

**3.** Verbindungen nach Anspruch 1, dadurch gekennzeichnet, daß $NR^1R^2$ eine n-Propylamino-, Isopropylamino-, tert.-Butylamino-, oder Piperidinogruppe ist,
der Rest $R^3$ ein Wasserstoffatom ist,
$R^4$ ein Wasserstoffatom oder eine Methoxygruppe ist, und n den Wert 1 oder 2 hat.

**4.** 1-(3,4-Dimethoxyphenyl)-3-[2'-(2-hydroxy-3-n-propylamino-propoxy)-phenyl]-1-propanon-hydrochlorid.

**5.** 1-(3-Methoxyphenyl)-3-[2'-(2-hydroxy-3-isopropylamino-propoxy)-phenyl]-1-propanon-hydrochlorid.

**6.** 1-Phenyl-3-[2'-(2-hydroxy-3-piperidino-propoxy)-phenyl]-1-propanon-hydrochlorid.

**7.** Verfahren zur Herstellung der Verbindungen nach Anspruch 1 und ihrer Säureadditionssalze, dadurch gekennzeichnet, daß man einen Phenoläther der allgemeinen Formel II

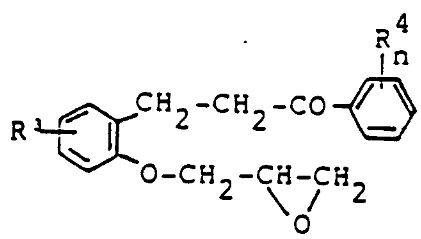

(II)

in der $R^3$, $R^4$ und n die in Anspruch 1 angegebene Bedeutung haben, mit einem Amin der allgemeinen Formel III

$HNR^1R^2$ (III)

in der $R^1$ und $R^2$ die in Anspruch 1 bis 6 angegebene Bedeutung haben, umsetzt und gegebenenfalls die erhaltene Verbindung mit einer Säure in ein Säureadditionssalz überführt.

**Claims**
**Claims for the following Contracting States : AT, BE, CH, LI, DE, FR, GB, IT, LU, NL, SE**

**1.** Aminopropanol derivatives of 3-(2'-hydroxyphenyl)-1-propanone compounds of the general formula I

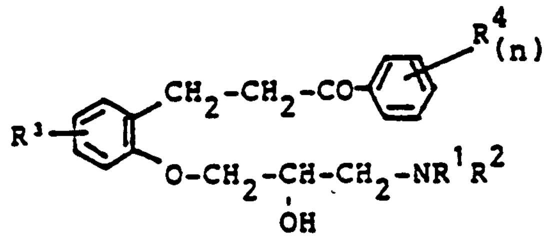

(I)

in which $R^1$ and $R^2$ are the same or different and are hydrogen atoms, alkyl, cycloalkyl, alkenyl, alkinyl or hydroxyalkyl residues with up to 6 C atoms each, alkoxyalkyl, alkylthioalkyl, or dialkylaminoalkyl residues with up to 9 C atoms each or phenylalkyl or phenoxyalkyl residues with up to 6 C atoms in the alkyl part, and wherein the phenyl residue is optionally substituted by an alkyl or alkoxy residue with up to 3 C atoms each or

$R^1$ and $R^2$ together with the nitrogen atom connecting them form a 5 to 7-membered saturated heterocyclic ring, which may optionally be substituted by one or two phenyl and/or hydroxy residues and may contain one oxygen or nitrogen atom as further heteroatom in the ring, wherein the additional nitrogen atom can be substituted by an alkyl residue with 1 to 3 C atoms or a phenyl residue,

$R^3$ is a hydrogen atom, an alkyl residue with up to 3 C atoms, a fluorine, chlorine, or bromine atom, a hydroxy group, an alkoxy group with up to 6 C atoms,

$R^4$ is a hydrogen atom, an alkyl residue with up to 3 C atoms, a fluorine, chlorine, or bromine atom, a hydroxy or an alkoxy group with up to 6 C atoms, and

n is an integer with a value from 1 to 5 and acid addition salts thereof.

**2.** Compounds according to Claim 1, characterized in that $NR^1R^2$ is a tert.-pentyl amino, n-propylamino, 1,1-dimethylpropylamino, morpholino, isopropylamino, tert.-butylamino, pyrrolidino, piperidino or cyclohexyl amino group,
the residue $R^3$ is a hydrogen atom, a methyl, methoxy or hydroxy group,
$R^4$ is a hydrogen atom, a methyl, methoxy, or hydroxy group, and
n has the value 1, 2 or 3.

**3.** Compounds according to Claim 1, characterized in that $NR^1R^2$ is a n-propylamino, isopropylamino, tert.-butylamino or piperidino group,
the residue $R^3$ is a hydrogen atom,
$R^4$ is a hydrogen atom or a methoxy group, and
n has the value 1 or 2.

**4.** 1-(3,4-dimethoxyphenyl)-3-[2'-(2-hydroxy-3-n-propylaminopropoxy)-phenyl]-1-propanone hydrochloride.

**5.** 1-(3-methoxyphenyl)-3-[2'-(2-hydroxy-3-isopropylaminopropoxy)-phenyl]-1-propanone hydrochloride.

**6.** 1-phenyl-3-[2'-(2-hydroxy-3-piperidino-propoxy)-phenyl]-1-propanone hydrochloride.

**7.** Process for producing compounds according to Claim 1 and the acid addition salts thereof, characterized in that a phenol ether of the general formula II

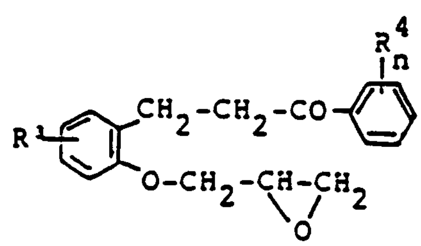

(II)

in which $R^3$, $R^4$ and n are as defined in Claim 1, is reacted with an amine of the general formula III

$HNR^1R^2$ (III)

in which $R^1$ and $R^2$ are as defined in Claims 1 to 6 and the compound obtained is optionally converted with an acid into an acid addition salt.

**8.** Pharmaceutical composition characterized by a content of a compound according to any one of Claims 1 to 6.

**Claims for the following Contracting State : ES**

**1.** Process for producing aminopropanol derivatives of 3-(2'-hydroxyphenyl)-1-propanone compounds of the general formula I

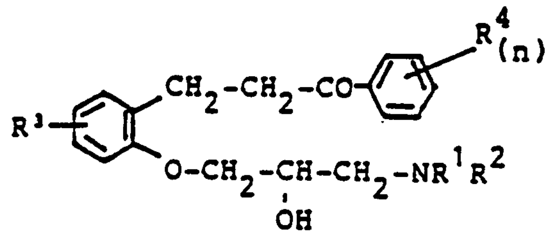

(I)

in which $R^1$ and $R^2$ are the same or different and are hydrogen atoms, alkyl, cycloalkyl, alkenyl, alkinyl or hydroxyalkyl residues with up to 6 C atoms each, alkoxyalkyl, alkylthioalkyl, or dialkylaminoalkyl residues with up to 9 C atoms each or phenylalkyl or phenoxyalkyl residues with up to 6 C atoms in the alkyl part, and wherein the phenyl residue is optionally substituted by an alkyl or alkoxy residue with up to 3 C atoms each or
$R^1$ and $R^2$ together with the nitrogen atom connecting them form a 5 to 7-membered saturated heterocyclic ring, which may optionally be substituted by one or two phenyl and/or hydroxy residues and may contain one oxygen or nitrogen atom as further heteroatom in the ring, wherein the additional nitrogen atom can be substituted by an alkyl residue with 1 to 3 C atoms or a phenyl residue,
$R^3$ is a hydrogen atom, an alkyl residue with up to 3 C atoms, a fluorine, chlorine, or bromine atom, a hydroxy group, an alkoxy group with up to 6 C atoms,
$R^4$ is a hydrogen atom, an alkyl residue with up to 3 C atoms, a fluorine, chlorine, or bromine atom, a hydroxy or an alkoxy group with up to 6 C atoms, and
n is an integer with a value from 1 to 5
and acid addition salts thereof, characterized in that a phenol ether of the general formula II

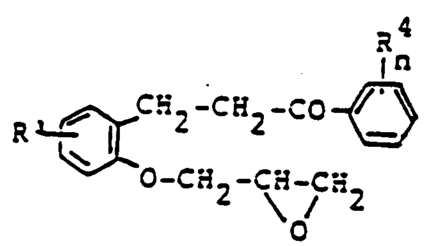

(II)

in which $R^3$, $R^4$ and n have the above meaning is reacted with an amine of the general formula III

$HNR^1R^2$ (III)

in which $R^1$ and $R^2$ have the above meaning and the compound obtained is optionally converted with an acid into an acid addition salt.

**2.** Process according to Claim 1, characterized in that $NR^1R^2$ is a tert.-pentyl amino, n-propylamino, 1,1-dimethylpropylamino, morpholino, isopropylamino, tert.-butylamino, pyrrolidino, piperidino or cyclohexyl amino group,
the residue $R^3$ is a hydrogen atom, a methyl, methoxy or hydroxy group,

$R^4$ is a hydrogen atom, a methyl, methoxy, or hydroxy group, and
n has the value 1, 2 or 3.

**3.** Process according to Claim 1, characterized in that $NR^1R^2$ is a n-propylamino, isopropylamino, tert.-butylamino or piperidino group,
the residue $R^3$ is a hydrogen atom,
$R^4$ is a hydrogen atom or a methoxy group, and
n has the value 1 or 2.

**4.** Process according to Claim 1 for producing 1-(3,4-dimethoxyphenyl)-3-[2'-(2-hydroxy-3-n-propylaminopropoxy)-phenyl]-1-propanone hydrochloride.

**5.** Process according to Claim 1 for producing 1-(3-methoxyphenyl)-3-[2'-(2-hydroxy-3-isopropylaminopropoxy)-phenyl]-1-propanone hydrochloride.

**6.** Process according to Claim 1 for producing 1-phenyl-3-[2'-(2-hydroxy-3-piperidino-propoxy)-phenyl]-1-propanone hydrochloride.

**Claims for the following Contracting State : GR**

**1.** Aminopropanol derivatives of 3-(2'-hydroxyphenyl)-1-propanone compounds of the general formula I

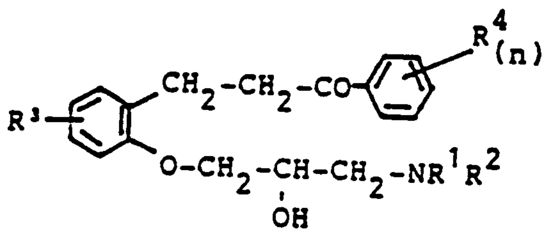

(I)

in which $R^1$ and $R^2$ are the same or different and are hydrogen atoms, alkyl, cycloalkyl, alkenyl, alkinyl or hydroxyalkyl residues with up to 6 C atoms each, alkoxyalkyl, alkylthioalkyl, or dialkylaminoalkyl residues with up to 9 C atoms each or phenylalkyl or phenoxyalkyl residues with up to 6 C atoms in the alkyl part, and wherein the phenyl residue is optionally substituted by an alkyl or alkoxy residue with up to 3 C atoms each or
$R^1$ and $R^2$ together with the nitrogen atom connecting them form a 5 to 7-membered saturated heterocyclic ring, which may optionally be substituted by one or two phenyl and/or hydroxy residues and may contain one oxygen or nitrogen atom as further heteroatom in the ring, wherein the additional nitrogen atom can be substituted by an alkyl residue with 1 to 3 C atoms or a phenyl residue,
$R^3$ is a hydrogen atom, an alkyl residue with up to 3 C atoms, a fluorine, chlorine, or bromine atom, a hydroxy group, an alkoxy group with up to 6 C atoms,
$R^4$ is a hydrogen atom, an alkyl residue with up to 3 C atoms, a fluorine, chlorine, or bromine atom, a hydroxy or an alkoxy group with up to 6 C atoms,
and n is an integer with a value from 1 to 5 and acid addition salts thereof.

**2.** Compounds according to Claim 1, characterized in that $NR^1R^2$ is a tert.-pentyl amino, n-propylamino, 1,1-dimethylpropylamino, morpholino, isopropylamino, tert.-butylamino, pyrrolidino, piperidino or cyclohexyl amino group,
the residue $R^3$ is a hydrogen atom, a methyl, methoxy or hydroxy group,
$R^4$ is a hydrogen atom, a methyl, methoxy, or hydroxy group, and
n has the value 1, 2 or 3.

**3.** Compounds according to Claim 1, characterized in that $NR^1R^2$ is a n-propylamino, isopropylamino, tert.-butylamino or piperidino group,
the residue $R^3$ is a hydrogen atom,
$R^4$ is a hydrogen atom or a methoxy group, and
n has the value 1 or 2.

**4.** 1-(3,4-dimethoxyphenyl)-3-[2'-(2-hydroxy-3-n-propylaminopropoxy)-phenyl]-1-propanone hydrochloride.

**5.** 1-(3-methoxyphenyl)-3-[2'-(2-hydroxy-3-isopropylaminopropoxy)-phenyl]-1-propanone hydrochloride.

**6.** 1-phenyl-3-[2'-(2-hydroxy-3-piperidino-propoxy)-phenyl]-1-propanone hydrochloride.

**7.** Process for producing compounds according to Claim 1 and the acid addition salts thereof, characterized in that a phenol ether of the general formula II

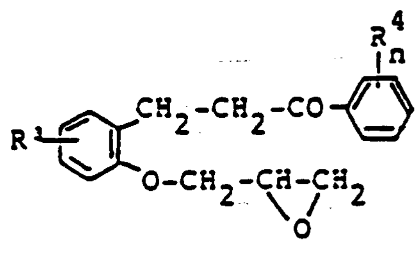

(II)

in which $R^3$, $R^4$ and n are as defined in Claim 1 is reacted with an amine of the general formula III

$HNR^1R^2$ (III)

in which $R^1$ and $R^2$ are as defined in Claims 1 to 6 and the compound obtained is optionally converted with an acid into an acid addition salt.

**Revendications**

**Revendications pour les Etats contractants suivants : AT, BE, CH, LI, DE, FR, GB, IT, LU, NL, SE**

**1.** Dérivés d'aminopropanol de composés de 3-(2'-hydroxyphényl)-1-propanone répondant à la formule générale I

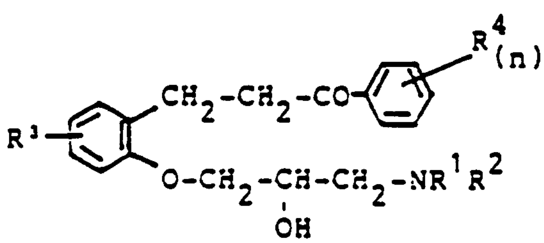

(I)

dans laquelle $R^1$ et $R^2$ sont identiques ou différents et représentent des atomes d'hydrogène, des radicaux alkyle, cycloalkyle, alcényle, alcynyle ou hydroxyalkyle comportant chacun jusqu'à 6 atomes de carbone, des radicaux alcoxyalkyle, alkylthioalkyle ou dialkylaminoalkyle comportant chacun jusqu'à 9 atomes de carbone, ou des radicaux phénylalkyle ou phénoxyalkyle comportant jusqu'à 6 atomes de carbone dans la fraction alkyle, le radical phényle étant éventuellement substitué par un radical alkyle ou alcoxy comportant chacun jusqu'à 3 atomes de carbone, ou $R^1$ et $R^2$ forment, conjointement avec l'atome d'azote qui les relie, un noyau hétérocyclique saturé pentagonal à heptagonal, qui peut éventuellement être substitué par un ou deux radicaux phényle et/ou hydroxy et qui peut contenir dans le noyau un atome d'oxygène ou d'azote comme autre hétéroatome, l'atome d'azote supplémentaire pouvant être substitué par un radical alkyle comportant 1 à 3 atomes de carbone ou par un radical phényle,
$R^3$ représente un atome d'hydrogène, un radical alkyle comportant jusqu'à 3 atomes de carbone, un atome de fluor, de chlore ou de brome, un groupe hydroxy ou un groupe alcoxy comportant jusqu'à 6 atomes de carbone,
$R^4$ représente un atome d'hydrogène, un radical alkyle comportant jusqu'à 3 atomes de carbone, un atome de fluor, de chlore ou de brome, un groupe hydroxy ou un groupe alcoxy comportant jusqu'à 6 atomes de carbone, et

n est un nombre entier ayant une valeur de 1 à 5,
et leurs sels d'addition d'acide.

2. Composés suivant la revendication 1, caractérisés en ce que $NR^1R^2$ représente un groupe tert.-pentylamino, n-propylamino, 1,1-diméthylpropylamino, morpholino, isopropylamino, tert.-butylamino, pyrrolidino, pipéridino ou cyclohexylamino, en ce que le radical R3 est un atome d'hydrogène ou un groupe méthyle, méthoxy ou hydroxy, en ce que $R^4$ représente un atome d'hydrogène, un groupe méthyle, méthoxy ou hydroxy, et en ce que n a la valeur de 1, 2 ou 3.

3. Composés suivant la revendication 1, caractérisés en ce que $NR^1R^2$ représente un groupe n-propylamino, isopropylamino, tert.-butylamino ou pipéridino, en ce que le radical R3 est un atome d'hydrogène, en ce que $R^4$ est un atome d'hydrogène ou un groupe méthoxy et en ce que n a la valeur de 1 ou de 2.

4. Chlorhydrate de 1-(3,4-diméthoxyphényl)-3-(2'-(2-hydroxy-3-n-propylamino-propoxy)-phényl)-1-propanone.

5. Chlorhydrate de 1-(3-méthoxyphényl)-3-(2'-(2-hydroxy-3-isopropylamino-propoxy)-phényl)-1-propanone.

6. Chlorhydrate de 1-phényl-3-(2'-(2-hydroxy-3-pipéridino-propoxy)-phényl)-1-propanone.

7. Procédé de préparation des composés suivant la revendication 1 et de leurs sels d'addition d'acide, caractérisé en ce qu'on fait réagir un phénoléther répondant à la formule générale II

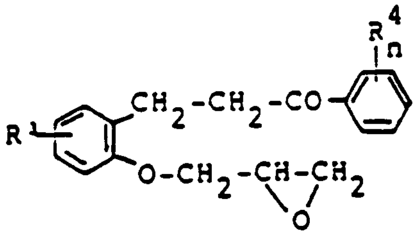

(II)

dans laquelle $R^3$, $R^4$ et n ont la signification indiquée dans la revendication 1, avec une amine répondant à la formule générale III

$HNR^1R^2$ (III)

dans laquelle $R^1$ et $R^2$ ont la signification indiquée dans les revendications 1 à 6, et en ce qu'avec un acide on convertit éventuellement le composé obtenu en un sel d'addition d'acide.

8. Médicament, caractérisé par une teneur en un composé suivant une des revendications 1 à 6.

**Revendications pour l'Etat contractant suivant : ES**

1. Procédé de préparation de dérivés d'aminopropanol de composés de 3-(2'-hydroxyphényl)-1-propanone répondant à la formule générale I

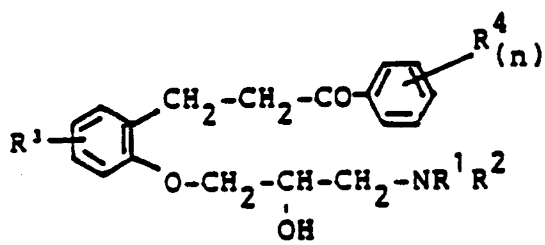

(I)

dans laquelle $R^1$ et $R^2$ sont identiques ou différents et représentent des atomes d'hydrogène, des radicaux alkyle, cycloalkyle, alcényle, alcynyle ou hydroxyalkyle comportant chacun jusqu'à 6 atomes de carbone, des radicaux alcoxyalkyle, alkylthioalkyle ou dialkylaminoalkyle comportant chacun jusqu'à 9 atomes de carbone, ou des radicaux phénylalkyle ou phénoxyalkyle comportant jusqu'à 6 atomes de carbone dans la fraction alkyle, le radical phényle étant éventuellement substitué par un radical alkyle ou alcoxy comportant chacun jusqu'à 3 atomes de carbone, ou $R^1$ et $R^2$ forment, conjointement avec l'atome d'azote qui les relie, un noyau hétérocyclique saturé pentagonal à heptagonal, qui peut éventuellement être substitué par un ou deux radicaux phényle et/ou hydroxy et qui peut contenir dans le noyau un atome d'oxygène ou d'azote comme autre hétéroatome, l'atome d'azote supplémentaire pouvant être substitué par un radical alkyle comportant 1 à 3 atomes de carbone ou par un radical phényle,

$R^3$ représente un atome d'hydrogène, un radical alkyle comportant jusqu'à 3 atomes de carbone, un atome de fluor, de chlore ou de brome, un groupe hydroxy ou un groupe alcoxy comportant jusqu'à 6 atomes de carbone,

$R^4$ représente un atome d'hydrogène, un radical alkyle comportant jusqu'à 3 atomes de carbone, un atome de fluor, de chlore ou de brome, un groupe hydroxy ou un groupe alcoxy comportant jusqu'à 6 atomes de carbone, et

n est un nombre entier ayant une valeur de 1 à 5,

et de leurs sels d'addition d'acide, caractérisé en ce qu'on fait réagir un phénoléther répondant à la formule générale II

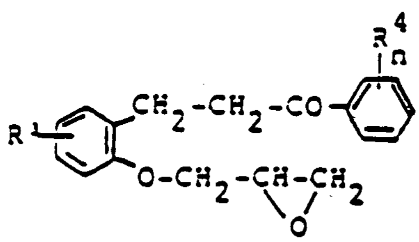

(II)

dans laquelle $R^3$, $R^4$ et n ont la signification indiquée précédemment, avec une amine répondant à la formule générale III

$HNR^1R^2$ (III)

dans laquelle $R^1$ et $R^2$ ont la signification indiquée précédemment, et en ce qu'avec un acide on convertit éventuellement le composé obtenu en un sel d'addition d'acide.

2. Procédé suivant la revendication 1, caractérisé en ce que $NR^1R^2$ représente un groupe tert.-pentylamino, n-propylamino, 1,1-diméthylpropylamino, morpholino, isopropylamino, tert.-butylamino, pyrrolidino, pipéridino ou cyclohexylamino, en ce que le radical R3 est un atome d'hydrogène ou un groupe méthyle, méthoxy ou hydroxy, en ce que $R^4$ représente un atome d'hydrogène, un groupe méthyle, méthoxy ou hydroxy, et en ce que n a la valeur de 1, 2 ou 3.

3. Procédé suivant la revendication 1, caractérisé en ce que $NR^1R^2$ représente un groupe n-propylamino, isopropylamino, tert.-butylamino ou pipéridino, en ce que le radical R3 est un atome d'hydrogène, en ce que $R^4$ est un atome d'hydrogène ou un groupe méthoxy et en ce que n a la valeur de 1 ou de 2.

4. Procédé suivant la revendication 1 pour la préparation de chlorhydrate de 1-(3,4-diméthoxyphényl)-3-(2'-(2-hydroxy-3-n-propylamino-propoxy)-phényl)-1-propanone.

5. Procédé suivant la revendication 1 pour la préparation de chlorhydrate de 1-(3-méthoxyphényl)-3-(2'-(2-hydroxy-3-isopropylamino-propoxy)-phényl)-1-propanone.

6. Procédé suivant la revendication 1 pour la préparation de chlorhydrate de 1-phényl-3-(2'-(2-hydroxy-3-pipéridinopropoxy)-phényl)-1-propanone.

**Revendications pour l'Etat contractant suivant : GR**

1. Dérivés d'aminopropanol de composés de 3-(2'-hydroxyphényl)-1-propanone répondant à la formule générale I

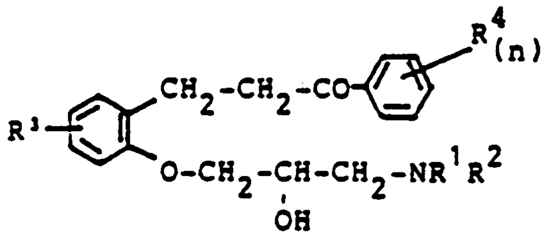

(I)

dans laquelle $R^1$ et $R^2$ sont identiques ou différents et représentent des atomes d'hydrogène, des radicaux alkyle, cycloalkyle, alcényle, alcynyle ou hydroxyalkyle comportant chacun jusqu'à 6 atomes de carbone, des radicaux alcoxyalkyle, alkylthioalkyle ou dialkylaminoalkyle comportant chacun jusqu'à 9 atomes de carbone, ou des radicaux phénylalkyle ou phénoxyalkyle comportant jusqu'à 6 atomes de carbone dans la fraction alkyle, le radical phényle étant éventuellement substitué par un radical alkyle ou alcoxy comportant chacun jusqu'à 3 atomes de carbone, ou $R^1$ et $R^2$ forment, conjointement avec l'atome d'azote qui les relie, un noyau hétérocyclique saturé pentagonal à heptagonal, qui peut éventuellement être substitué par un ou deux radicaux phényle et/ou hydroxy et qui peut contenir dans le noyau un atome d'oxygène ou d'azote comme autre hétéroatome, l'atome d'azote supplémentaire pouvant être substitué par un radical alkyle comportant 1 à 3 atomes de carbone ou par un radical phényle,
$R^3$ représente un atome d'hydrogène, un radical alkyle comportant jusqu'à 3 atomes de carbone, un atome de fluor, de chlore ou de brome, un groupe hydroxy ou un groupe alcoxy comportant jusqu'à 6 atomes de carbone,
$R^4$ représente un atome d'hydrogène, un radical alkyle comportant jusqu'à 3 atomes de carbone, un atome de fluor, de chlore ou de brome, un groupe hydroxy ou un groupe alcoxy comportant jusqu'à 6 atomes de carbone, et
n est un nombre entier ayant une valeur de 1 à 5,
et leurs sels d'addition d'acide.

2. Composés suivant la revendication 1, caractérisés en ce que $NR^1R^2$ représente un groupe tert.-pentylamino, n-propylamino, 1,1-diméthylpropylamino, morpholino, isopropylamino, tert.-butylamino, pyrrolidino, pipéridino ou cyclohexylamino, en ce que le radical R3 est un atome d'hydrogène ou un groupe méthyle, méthoxy ou hydroxy, en ce que $R^4$ représente un atome d'hydrogène, un groupe méthyle, méthoxy ou hydroxy, et en ce que n a la valeur de 1, 2 ou 3.

3. Composés suivant la revendication 1, caractérisés en ce que $NR^1R^2$ représente un groupe n-propylamino, isopropylamino, tert.-butylamino ou pipéridino, en ce que le radical R3 est un atome d'hydrogène, en ce que $R^4$ est un atome d'hydrogène ou un groupe méthoxy et en ce que n a la valeur de 1 ou de 2.

4. Chlorhydrate de 1-(3,4-diméthoxyphényl)-3-(2'-(2-hydroxy-3-n-propylamino-propoxy)-phényl)-1-propanone.

5. Chlorhydrate de 1-(3-méthoxyphényl)-3-(2'-(2-hydroxy-3-isopropylamino-propoxy)-phényl)-1-propanone.

6. Chlorhydrate de 1-phényl-3-(2'-(2-hydroxy-3-pipéridino-propoxy)-phényl)-1-propanone.

7. Procédé de préparation des composés suivant la revendication 1 et de leurs sels d'addition d'acide, caractérisé en ce qu'on fait réagir un phénoléther répondant à la formule générale II

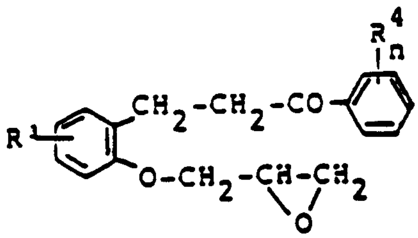

(II)

dans laquelle $R^3$, $R^4$ et n ont la signification indiquée dans la revendication 1, avec une amine répondant à la formule générale III

$HNR^1R^2$ (III)

dans laquelle $R^1$ et $R^2$ ont la signification indiquée dans les revendications 1 à 6, et en ce qu'avec un acide on convertit éventuellement le composé obtenu en un sel d'addition d'acide.